# FASCICULE DE BREVET EUROPEEN

(11) **EP 2 203 417 B1**
(45) Date de publication et mention de la délivrance du brevet: **06.11.2013**
(21) Numéro de dépôt: 08845202.4
(22) Date de dépôt: 28.10.2008
(51) Int. Cl.: C07C 255/03, C07C 255/07, A61L 9/01, A61K 8/40, A61Q 13/00, C11B 9/00, C11D 3/50

(54) **OCTANE(ÈNE) NITRILES SUBSTITUÉS, LEURS PROCÉDÉS DE SYNTHÈSES ET LEURS UTILISATIONS EN PARFUMERIE**
SUBSTITUIERTE OCTAN(EN)NITRILE, VERFAHREN ZU DEREN SYNTHESE UND DEREN ANWENDUNGEN IN DER DUFTSTOFFHERSTELLUNG
SUBSTITUTED OCTANE(ENE) NITRILES, METHODS FOR THE SYNTHESIS THEREOF AND USES THEREOF IN PERFUMERY

(30) Priorité: 29.10.2007 FR 0707587
(43) Date de publication de la demande: 07.07.2010
(73) Titulaire: V. Mane Fils, 06620 Bar sur Loup (FR)
(72) Inventeur: MANE, JEAN, 06130 Grasse (FR); CLINET, Jean-Claude, 06270 Villeneuve-Loubet (FR)
(74) Mandataire: Rançon, Xavier Lucien Abel
(86) Numéro de dépôt international: PCT/FR2008/051939
(87) Numéro de publication internationale: WO 2009/056756

(56) Documents cités:
- EP-A- 0 347 596
- WO-A-2006/077305

## Description

La présente invention concerne le domaine des fragrances, et reporte en particulier la préparation de nouveaux composés, leur procédé de synthèse et leur utilisation en parfumerie du fait de leurs propriétés odorantes. Les composés de l'invention sont des trimethyl-3,5,7-octane(ène) nitriles et leurs dérivés α-substitués et l'utilisation de ces composés en parfumerie notamment en tant que matière première. La présente invention concerne également des parfums et produits parfumés contenant lesdits composés.

De nombreux nitriles, aliphatiques ou aromatiques, sont actuellement utilisés dans l'art de la parfumerie. Ils ont été développés tant pour leur bonne stabilité dans les milieux agressifs que du fait de leur fréquente similarité olfactive avec les aldéhydes correspondants; ces derniers étaient utilisés en parfumerie bien avant le développement des nitriles. A titre indicatif, le brevet EP0347596 décrit des nitriles aliphatiques porteurs d'un groupe méthyle en position 5 ainsi que leur utilisation en parfumerie. Pour autant ces composés ne sont pas associés aux notes olfactives des composés de la présente invention.

Il a été identifié par la Demanderesse que certains trimethyl-3,5,7-octane(ène) nitriles et de leurs dérivés portant un substituant en position α du groupe nitrile de formule générale (I) représentée ci-dessous possèdent un intérêt réel en tant que fragrance, parfum, ou comme matière première pour des parfums ou des compositions parfumées. Les composés de l'invention ne sont pas seulement nouveaux dans l'art de la parfumerie mais à la connaissance de la Demanderesse, ils n'ont jamais été reportés en tant que tels dans la littérature. Or, l'industrie des parfums est de plus en plus soumise à des réglementations, et recherche des composés pouvant venir se substituer, ou s'identifier, à certains composés récemment interdits d'utilisation pour des raisons de sécurité ou de toxicité.

L'invention a notamment pour objet les composés de formule générale (I) ci-dessous : 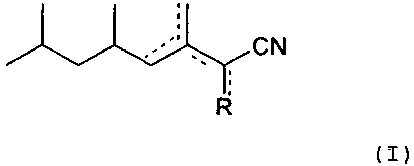 dans laquelle R est un atome d'hydrogène, un groupe méthyle ou un groupe méthylène ; et dans laquelle au plus une des quatre lignes pointillées représente une double liaison carbone-carbone.

Selon un mode de réalisation particulier de l'invention, la chaîne principale est saturée et R représente un atome d'hydrogène, un groupe méthyle ou un groupe méthylène.

Suivant un autre mode de réalisation préféré, le composé selon l'invention répond à la formule (II) suivante : 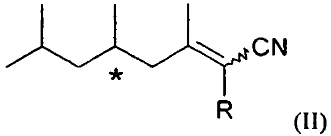 dans laquelle R est un atome d'hydrogène ou un groupe méthyle.

Suivant un autre mode de réalisation préféré, le composé selon l'invention répond à la formule (III) suivante : 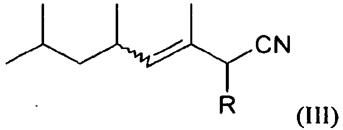 dans laquelle R est un atome d'hydrogène ou un groupe méthyle.

Suivant un autre mode de réalisation préféré, le composé selon l'invention répond à la formule (IV) suivante : 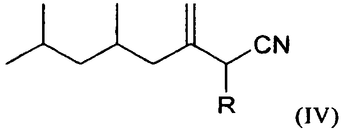 dans laquelle R est un atome d'hydrogène ou un groupe méthyle.

Suivant un autre mode de réalisation préféré, le composé selon l'invention répond à la formule (V) suivante : 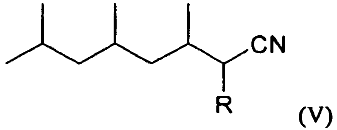 dans laquelle R est un atome d'hydrogène ou un groupe méthyle.

Suivant un autre mode de réalisation préféré, le nitrile ester insaturé répondant à la formule (VI) suivante est un des intermédiaires réactionnels pour la synthèse des composés selon l'invention:

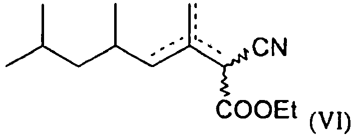

Suivant un autre mode de réalisation préféré, le beta-hydroxy-nitrile répondant à la formule (VII) suivante est un des intermédiaires réactionnels pour la synthèse des composés selon l'invention:

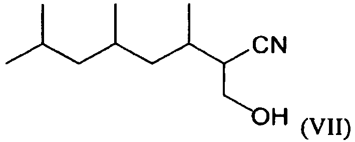

Suivant un autre mode de réalisation préféré, le composé selon l'invention répond à la formule (VIII) suivante :

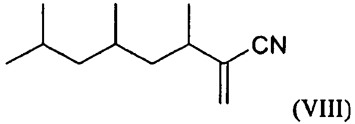

Suivant un autre mode de réalisation préféré, le composé selon l'invention répond à la formule (IX) suivante :

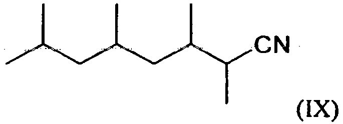

Les composés selon l'invention comportent un ou plusieurs carbones asymétriques et de ce fait peuvent être sous la forme de mélanges d'isomères optiques, notamment d'énantiomères et de diastéréoisomères. La présente invention entend protéger des compositions comprenant au moins un composé de formule générale (I), sous forme d'un isomère ou d'un mélange d'isomères, en particulier d'un énantiomère ou d'un mélange d'énantiomères, ou d'un mélange racémique, ou d'un diastéréoisomère ou mélange de diastéréoisomères. L'invention a donc également pour objet l'utilisation des composés de formule générale (I) comme agents odorants.

Ainsi, l'invention a pour objet une composition, caractérisée en ce qu'elle comprend au moins un composé selon l'invention sous forme d'un isomère ou d'un mélange d'isomères, d'un énantiomère ou d'un mélange d'énantiomères, ou d'un mélange racémique, ou d'un diastéréoisomère ou d'un mélange de diastéréoisomères. Suivant un mode de réalisation préféré, la composition selon l'invention comprend un mélange des composés de formule (II), (III), (IV) tels que définis ci-dessus. Suivant un autre mode de réalisation de l'invention, la composition comprend au moins un des deux isomères Z et E du composé (II) tel que défini ci-dessus ou un mélange desdits deux isomères. Suivant encore un autre mode de réalisation préféré, la composition comprend au moins un des quatre diastéréoisomères du composé (V) tel que défini ci-dessus ou un mélange desdits quatre diastéréoisomères.

L'invention a également pour objet une composition comprenant un mélange de deux isomères Z/E d'au moins un composé de formule (II), (III) et/ou (IV).

Les composés de l'invention ayant au moins un carbone asymétrique, l'invention a également pour objet une composition comprenant un mélange d'énantiomères ou de diastéréoisomères du composé (I) ; l'invention a également pour objet une composition comprenant un mélange d'énantiomères du composé (II) ; l'invention a également pour objet une composition comprenant un mélange d'énantiomères ou de diastéréoisomères du composé (III) ; l'invention a également pour objet une composition comprenant un mélange d'énantiomères ou de diastéréoisomères du composé (IV) ; l'invention a également pour objet une composition comprenant un mélange d'énantiomères ou de diastéréoisomères du composé (V) ; l'invention a également pour objet une composition comprenant un mélange d'énantiomères ou de diastéréoisomères du composé (VIII) ; l'invention a également pour objet une composition comprenant un mélange d'énantiomères ou de diastéréoisomères du composé (IX).

L'invention a également pour objet une composition comprenant un seul énantiomère d'un seul isomère Z ou E du composé (II) ; une composition comprenant un seul énantiomère ou diastéréoisomère d'un seul isomère Z ou E du composé (III) ; une composition comprenant un seul énantiomère ou diastéréoisomère du composé (IV) ; une composition comprenant un seul énantiomère ou diastéréoisomère du composé (V) ; une composition comprenant un seul énantiomère ou diastéréoisomère du composé (VIII) ; une composition comprenant un seul énantiomère ou diastéréoisomère du composé (IX).

Les composés de l'invention peuvent être préparés par de nombreuses méthodes connues de l'homme de l'art, en particulier l'invention a également pour objet un procédé de préparation des composés de l'invention, par une condensation de type Knoevenagel ²⁾ entre une cétone notamment la diméthyl-4,6 heptanone-2 et l'acide cyanoacétique ou ses esters ; de préférence, cette condensation est suivie de décarboxylations, tel que représenté, à titre illustratif uniquement, dans le schéma A. Il est bien entendu que le schéma A représente un procédé de synthèse des composés de l'invention dans lesquels R est un atome d'hydrogène.

La diméthyl-4,6- heptanone-2 est aisément accessible, par exemple à partir de sous-produits de la cétolisation alcaline de l'acétone à l'échelle industrielle³⁾. Trois isomères de position dont deux peuvent être cis ou trans, ou un mélange de ceux-ci, peuvent être obtenus dans la condensation, lesdits isomères se distinguant les uns des autres par la position relative du groupe nitrile et de la double liaison carbone-carbone crée ainsi que de la géométrie autour de ladite double liaison (α, β : isomères *E* et *Z* ; β, γ isomères *E* et *Z* ; β : méthylène).

Les quantités obtenues des différents isomères en mélange peuvent varier en fonction des conditions opératoires²⁾. Ainsi, de manière classique, l'accroissement du temps et de la température de l'étape de décarboxylation élève le taux des isomères conjugués (II), tandis qu'une réduction de la quantité de catalyseurs basiques favorise la formation des isomères β, γ insaturés (III *E* et *Z* , IV). Les composés (III) et (IV) peuvent être séparés du mélange par distillation en présence d'un acide fort alors qu'un traitement en présence de potasse alcoolique permet d'isoler les isomères α, β insaturés, purs (II).

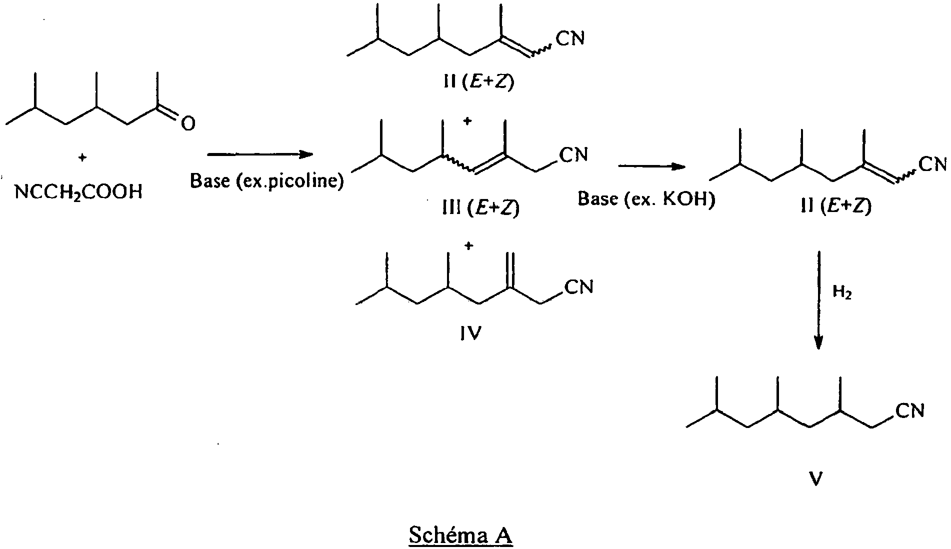

La réduction, par exemple par hydrogénation catalytique à température et pression ordinaire, des isomères (II), dénommés Verbetryle™, sur du charbon palladié conduit au nitrile saturé (V) avec un rendement de 88%. Le composé (V) est dénommé Dihydroverbetryle™ (schéma A).

Les composés selon l'invention se caractérisent tous par une note olfactive « hespéridés, verte » intense, avec pour chacun d'entre eux des spécificités : que le mélange des isomères II, III et IV dans des proportions (30(II) :70 (III+IV)) fait montre d'une note fruitée (poire), le Verbetryle™ obtenu par traitement basique du précédant se caractérise plutôt par des facettes irisées, fleuries un peu menthées.

Le Dihydroverbetryle™ (V) complète sa palette olfactive par des notes fleuries, aldéhydées rappelant le géranonitrile. Une touche menthée est également perceptible.

La substitution de l'acide cyanoacétique par son ester éthylique dans la condensation de Knoevenagel conduit au nitrile-ester insaturé (VI) (schéma B). La réduction de ce dernier, par exemple à l'aide de borohydrure de sodium conduit au β-hydroxy-nitrile (VII). Ce dernier permet l'élaboration du nitrile α, β-insaturé (VIII) qui génère le nitrile (IX) par hydrogénation dans les mêmes conditions que précédemment.

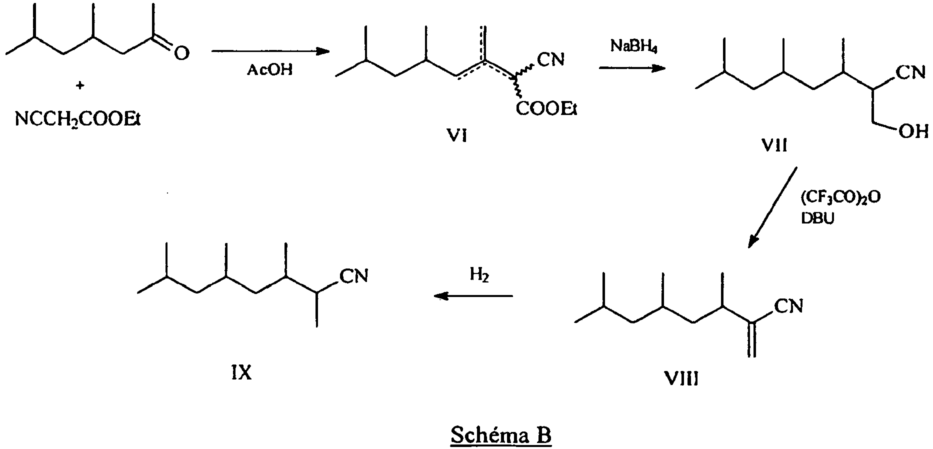

Le nitrile insaturé (VIII) se caractérise par des notes de type « Essence de mandarine, fleuries » tandis que son homologue saturé (IX) présente des notes « lactonée, fruitée et amande douce ».

Les composés selon l'invention sont des matières premières intenses sur le plan olfactif qui peuvent donc trouver un usage pour fournir, renforcer, voire améliorer la fragrance d'une large variété de produits.

L'invention a donc également pour objet l'utilisation d'au moins un composé de formule (I) selon l'invention comme agent odorant, comme agent de masquage d'odeur ou comme agent de neutralisation d'odeur, seul ou en en mélange avec un ou plusieurs autres composés odorants connus de l'homme du métier, que l'homme du métier est à même de choisir en fonction de l'effet recherché. Le ou les agents odorants supplémentaires peuvent être des composés de formule (I) ou d'autres agents odorants connus de l'homme du métier.

Au sens de la présente invention, le terme parfumerie désigne non seulement la parfumerie au sens habituel du terme, mais également les autres domaines dans lesquels l'odeur des produits est importante. Les composés de l'invention peuvent faire partie de compositions de parfumerie au sens habituel du terme, telles que bases et concentrés parfumants, eaux de Cologne, eaux de toilette, parfums et produits similaires ; de compositions topiques - en particulier cosmétiques telles que crèmes pour le visage et le corps, poudres de talc, produits d'hygiène corporelle notamment shampoings, produits lavants pour le corps et les cheveux, huiles pour cheveux, lotions capillaires, sels et huiles de bain, gels de douche et de bain, savons de toilette, anti-transpirants et désodorisants corporels, lotions et crèmes de rasage ou après-rasage, crèmes, dentifrices, bains de bouche, pommades, et produits similaires ; et de produits d'entretien, tels qu'assouplissants, adoucissants textiles, détergents, lessives, désodorisants d'ambiance, produits d'entretien et de désinfection de l'habitat, et produits similaires.

Les composés de l'invention sont donc des composés odorants, utiles en parfumerie, et notamment en tant que constituants d'un parfum. Dans le cadre de cette invention, on entend par parfum un mélange de substances odorantes qui peuvent être mises en solution ou mélangées à un support solide afin d'apporter l'odeur désirée à la peau ou à tout autre support pour lequel une odeur agréable est nécessaire ou souhaitable. Le terme odorant est utilisé ici pour désigner un composé qui exhale une odeur.

L'invention a donc pour objet une composition de parfumerie, notamment base ou concentré parfumant, eau de Cologne, eau de toilette ou parfum, caractérisée en ce qu'elle comprend au moins un composé selon l'invention. L'invention a en particulier pour objet une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade, caractérisée en ce qu'elle comprend au moins un composé selon l'invention. L'invention a également pour objet un produit d'entretien, notamment assouplissant, détergent, lessive, désodorisant d'ambiance, caractérisé en ce qu'il comprend au moins un composé selon l'invention. Un autre objet de l'invention est une méthode de traitement ou de soin cosmétique, préventive ou non, mettant en oeuvre au moins un composé de formule (I) ou au moins une composition comprenant au moins un composé de formule (I).

L'invention concerne également des compositions comprenant au moins un composé de l'invention et au moins une autre susbstance odorante, notamment choisie parmi les produits naturels que sont les huiles essentielles, les absolus, les résinoïdes, les concrètes, etc., mais aussi les produits synthétiques tels que les hydrocarbures, les alcools, les aldéhydes, les cétones, les éthers, les esters, les acétals, les nitriles, aliphatiques aromatiques ou hétérocycliques.

Les composés de l'invention peuvent être utilisés en parfumerie, notamment dans les parfums ou les compositions parfumées, dans une vaste gamme de concentration qui dépend autant de la nature du produit fini que de la concentration des autres ingrédients de la composition.

De préférence, les composés selon l'invention seront présents dans les compositions parfumantes ou les parfums, à une concentration d'au moins 0.001% en poids par rapport au poids total de la composition : à cette concentration, son odeur est perceptible. De préférence, les composés selon l'invention seront présents dans les compositions parfumantes ou les parfums, à une concentration d'au moins 0.01% en poids par rapport au poids total de la composition. Avantageusement, les composés selon l'invention seront présents dans les parfums en quantité d'au moins 1 ppm en poids et préférablement de 10 ppm.

L'invention concerne encore l'utilisation d'au moins un composé selon l'invention comme agent odorant ou comme agent de masquage d'odeur ou comme agent de neutralisation d'odeur, optionnellement en association avec d'autres agents odorants.

Les exemples suivants s'efforcent de présenter, à titre illustratif uniquement, la préparation et l'usage des composés de l'invention ; l'invention n'est en aucun cas limitée auxdits exemples.

### Exemple 1 : synthèse

### 1.1 Préparation du mélange de trimethyl-3,5,7 octène nitriles de formules (II), (III) et (IV) dans lesquelles R est un atome d'hydrogène.

La réaction est conduite dans un ballon tricol de 2 litres muni d'un thermomètre plongeant, d'un réfrigérant ascendant équipé d'un séparateur de phases et d'une ampoule de coulée isobare de 500 millilitres.

Dans le ballon sont introduits successivement 10g d'acétate d'ammonium (0.13 mole) 20,4 g de 2-picoline (0.22 mole), 160g de toluène et 568g de diméthyl-4,6 heptanone-2 (4 moles). L'ensemble est porté au reflux total alors que l'eau qui se sépare est éliminée au fur et à mesure.

Une solution de 170 g d'acide cyanoacétique (2 moles) dans 70g d'eau claire (solution à 70 % massique) est versée dans l'ampoule de coulée. Cette solution est ajoutée régulièrement en 12 heures dans le ballon. Des mousses se forment dues à l'élimination d'eau et de dioxyde de carbone. L'eau est éliminée régulièrement du séparateur de phases. Le reflux total est maintenu 6 heures après la fin de l'addition puis l'absence de dégagement de dioxyde de carbone est vérifiée. Le distillat étant clair, il est collecté dans une recette et mis à part. L'opération est interrompue lorsqu'on obtient une température de 145-150°C dans les vapeurs. Le chauffage est interrompu tandis qu'un montage de distillation classique est substitué au séparateur de phases. Une pompe à anneau liquide est connectée au montage résultant puis le vide est décru très progressivement. Le second distillat est collecté au fur et à mesure jusqu'à atteindre une température vapeur de 60-65°C sous 2400 Pa. Il s'agit de l'excès de cétone introduit.

Le résidu est lavé avec une solution aqueuse saturée de bicarbonate de sodium, séché puis il est distillé (Eb = 87-95°C / 667 Pa). 210 g de nitrile sont collectés ce qui représente un rendement de 63.5%.
La proportion des isomères conjugués (E+Z) (II) par rapport aux isomères non conjugués, (III) et (IV), (voir schéma A) a été établie par intégration de signaux spécifiques en RMN du proton (signaux en alpha du nitrile) : pour les isomères (II), le proton vinylique est situé à 5.0 ppm pour un isomère et à 5.18 ppm pour l'autre, les protons localisés en α du groupe nitrile sont situés à 3.05 ppm pour les isomères (III) et (IV) pour lesquels R est un atome d'hydrogène.
Ainsi peut-on déduire que les isomères (I) représentent 30% du mélange.

### 1.2 Préparation du trimethyl-3,5,7 octèn-2 nitrile de formule II dans laquelle R est un atome d'hydrogène: (Verbetryle™).

Dans un ballon de 500 mL, sont introduits successivement, 100g d'éthanol, 16.56g de potasse caustique (0.295 mole) puis 210g de nitrile à équilibrer (1.27 mole). Le mélange est agité 16 heures à température ambiante puis il est refroidi entre 0 et 5°C. De l'acide phosphorique à 85% (54,4g) est additionné lentement, le pH est contrôlé (il doit être à peu près égal à 7) puis l'éthanol est distillé sous pression réduite.

Le résidu est rectifié sous vide à travers une colonne courte puis il est distillé sous vide (Eb = 85-90 °C / 667 Pa). Le produit distillé pèse 196g, soit un rendement de 93.3%. Ce produit est un mélange de 2 isomères *E* : *Z* dans des proportions respectives de 60 :40 (Chromatographie Phase Vapeur). Il est obtenu sous la forme d'un liquide incolore doté d'une odeur très caractéristique.
1) IR : 3051, 2956, 2218, 1629, 1459, 1385, 1367, 829, 806, 791 cm⁻¹
2) MS (70eV) : m/e= 165(M+˙), 164, 151, 150, 122, 108, 85, 81, 69 uma
3) RMN ¹H : CDCl₃ ; 200 MHz :
   δ(ppm) : 0.80 à 0.90( m, 9H) ; 1.04(d*d , 7.0/ 7.0 Hz, 2H) ; 1.12(d*d , 7.3/ 7.3 Hz, 2H) ; 1.50 à 2.00 (m, 4H) ; 1.92 (d, 1.6 Hz, 3H) ; 2.02 (d, 1.0 Hz, 3H) ; 2.10 à 2.40(m, 4H) ; 5.10 (m,1H) ; 5.18 (m, 1H)
   *Les signaux soulignés sont relatifs à l'isomère minoritaire.*
4) RMN ¹³C : CDCl₃ ; 50MHz :
   δ(ppm) :19.3 ;19.4 ;20.8 ;22.0 ;22.1 ;23.0 ;23.3 ;23.5 ;2 5.1 ;44.1 ;46.2 ;46.3 ;46.9 ;96.3 ;96.7 ;117.2 ;169.5 ; 164.6

### 1.3 Préparation du trimethyl-3,5,7 octane nitrile (V) (Dihydroverbetryle)

Dans un autoclave de 250 mL, sont introduits successivement 16.5 g de Verbetryle (II), 2g de palladium sur charbon (5%) puis 150 mL de toluène sec. La solution résultante est agitée 24 heures sous 1 atmosphère d'hydrogène, filtrée puis lavée avec une solution 5N d'acide chlorhydrique. Le produit obtenu est distillé sous vide (Eb=92-95°C / 800 Pa) et conduit à 15g de nitrile V.

Le rendement est de 90%.
1) IR : 2249, 1460, 1420, 1386, 1368 cm⁻¹
2) MS (70 eV) : m/e= 167 (M+˙) ; 166 ; 152 ; 129 ; 110(100) ; 96 ; 85 ; 69 uma
3) RMN ¹H CDCl₃ 200 MHz : *Mélange de 4 diastéréoisomères.*
   δ (ppm) :0.8 à 0.9 (m, 3H) ; 1.0 à 1.1(m, 3H) ; 1.10 à 2.00 (m, 7H) ; 2.20 à 2.35(m, 2H).
4) RMN ¹³C : CDCl₃ ; 50MHz :
   δ(ppm) : 17.2 ; 17.5 ; 18.1 ; 18.2 ; 20.2 ; 20.4 ; 21.4 ; 21.6 ; 22.4 ; 23.1 ; 23.2 ; 23.4 ; 25.7 ; 25.8 ; 25.9 ; 26.0 ; 41.9 ; 42.0 ; 44.6 ; 45.2 ; 94.8 ; 116.9 ; 117.0 .

### 1.4 Préparation du carboéthoxy-2 trimethyl-3,5,7 octèn-2 nitrile (VI) :

Dans un ballon de 250 mL, équipé d'un montage de Dean-Stark sont introduits successivement 25.6 g de dimethyl-4,6 heptanone (0.18 mole), 14.34 g de cyanoacétate d'éthyle (0.127 mole), 1.95 g d'acétate d'ammonium, 6 g d'acide acétique et 100 mL de méthylcyclohexane. Le mélange est porté au reflux en séparant l'eau formée au fur et à mesure. Après une heure le mélange est refroidi à température ambiante puis il est lavé à neutralité avec une solution saturée de bicarbonate de sodium. Le résidu est distillé sous vide (800 Pa) afin de récupérer la cétone en excès (6.5g) puis sous haut vide afin de collecter le cyanoester VI. 25.8g d'un liquide incolore sont collectés (Eb =100-104°C / 26 Pa), soit un rendement de 85%.

L'examen de spectre de RMN du proton (voir ci-dessous) montre l'absence de proton vinylique et donc de dérivés déconjugués. L'intégration des signaux des radicaux méthyles portés par la double liaison carbone-carbone (2.28 ppm pour l'isomère minoritaire, 2.36 pour le majoritaire) montre la présence de deux isomères E/Z dans des proportions 40/60.
1) IR: 2224, 1731, 1603, 1465, 1368, 1287, 1228, 1099, 1070, 858, 777 cm⁻¹
2) MS (70 eV) : m/e= 237 (M+˙), 222, 192, 180, 164, 153, 135, 125, 97, 85(100), 69 uma .
3) RMN ¹H : CDCl₃ ; 200 MHz : *Les signaux soulignés correspondent à l'isomère minoritaire.* δ(ppm) : 0.8 à 1.0 (m,9H), 1.0 à 1.3 (m, 2H), 1.34 (t, J=7.2Hz, 3H), 1.35 (t, J=7.2Hz, 3H), 1.5 à 2.1 (m, 2H), 2.28 (s, 3H), 2.36 (s, 3H), 2.48 (m, 1H), 2.76 (m, 1H), 4.27 (q ., J=7.2Hz, 2H), 4.28 (q., J=7.2Hz, 2H)
4) RMN ¹H CDCl₃ ; 200 MHz: *Les signaux soulignés correspondent à l'isomère minoritaire*
   δ(ppm) :14.8 ; 19.7 ; 21.6 ; 22.4 ; 23.6 ; 23.7 ; 25.5 ; 25.7 ; 30.4 ; 30.9 ; 42.6 ; 46.7 ; 46.9 ; 48.6 ; 62.0 ; 106.0 ; 106.4 ; 116.3 ; 162.1 ; 162.4 ; 177.0 ; 177.1.

### 1.5 Préparation de l'hydroxyméthyl-2 trimethyl-3,5,7 octane nitrile (VII) :

La réaction est mise en oeuvre dans un ballon tricol de 1 L, équipé d'un réfrigérant ascendant, d'un thermomètre plongeant et d'une ampoule de coulée isobare de 250 mL. Dans le ballon sont introduits successivement 11.4g (0.3 mole) de borohydrure de sodium puis 300 mL d'éthanol absolu refroidi à 0°C. Dans l'ampoule de coulée une solution de 47.4g (0.2 mole) de cyanoester (VI) dans 100 mL d'éthanol absolu est versée. Le contenu du ballon est porté à -15°c puis le contenu de l'ampoule est additionné sans dépasser 0°C. Le mélange résultant est agité 16 heures à température ambiante, puis il est traité successivement avec 50 mL d'acétone et 200 mL d'acide chlorhydrique 5N. Le mélange est agité 5 heures puis l'éthanol est éliminé par distillation. Le résidu est extrait avec 3 fois 200 mL d'éther de méthyle et de *t*-butyle. Les phases organiques sont concentrées puis distillées sous haut vide. Un liquide incolore est obtenu (30.7g) (Eb=100-102 °C / 26 Pa) ce qui représente un rendement de 78%.
1) IR : 3460, 2244, 1465, 1385, 1367, 1166, 1062, 1028, 573 cm⁻¹
2) MS (70 eV): m/e= 197 (M+˙), 182, 152, 141, 140, 124, 110, 85, 68, 43(100) uma.
3) RMN ¹H CDCl₃ ; 200 MHz: *Mélange de 4 diastéréoisomères*
   δ(ppm) :0.8 à 0.9 (m, 3H) ; 1.0 à 1.1(m, 3H) ; 1.10 à 2.00 (m, 7H) ; 2.60 à 2.90(m, 1H) ; 3.50(s,1H) ; 3.60 à 4.00(m,2H).
4) RMN ¹³C : CDCl₃ ; 50MHz : *Mélange de 4 diastéréoisomères*
   δ(ppm) : 13.6 ; 19.3 ; 15.5 ; 15.9 ; 16.9 ; 17.2 ; 17.9 ; 18.6 ; 19.5 ; 20.0 ; 20.2 ; 20.4 ; 20.9 ; 21.2 ; 21.4 ; 22.0 : 22.9 ; 23.0 ; 23.1 ; 25.2 ; 25.3 ; 25.4 ; 25.6 ; 26.7 ; 26.9 ; 27.4 ; 27.5 ; 38.1 ; 38.4 ; 39.5 ; 39.6 ; 39.7 ; 40.1 ; 41.0 ; 41.2 ; 43.3 ; 44.3 ; 45.1 ; 45.6 ; 58.4 ; 58.5 ; 59.0 ; 59.2 ; 117.7 ; 117.9 ; 118.5 ; 118.6.

### 1.6 Préparation du méthylèn-2 trimethyl-3,5,7 octane nitrile (VIII) :

Dans un ballon tricol de 250 mL, sec et purgé à l'azote sont introduits successivement 19.7g du cyanoalcool (VII) (0.1 mole) ,150 mL de chlorure de méthylène anhydre, puis 18.26 g (0.11mole) de DBU (1,8-diazabicyclo[5.4.0]undec-7-ene). La solution est portée à 0°C puis 22.05g (0.105 mole) d'anhydride trifluoroacétique sont additionnés goutte à goutte sans dépasser 5°C dans la masse. La solution obtenue est agitée 16 heures à température ambiante puis elle est hydrolysée, séchée et concentrée. Le résidu est distillé sous vide (800 Pa) pour fournir 16 g de nitrile insaturé (VIII) distillant à 105-108°C. Le rendement est de 89%.
1) IR : 2222, 1622, 1460, 1383, 935, 651 cm⁻¹
2) MS (70 eV) : m/e= 179 (M+˙), 178, 164, 136, 122, 108, 95, 83, 57(100) uma.
3) RMN ¹H : CDCl₃ ; 200 MHz : *Mélange de 2 diastéréoisomères.*
   δ (ppm) :0.8 à 0.9 (m, 3H) ; 1.00 à 1.80 (m, 9H) ; 2.5 (m, 1H) ; 5.70 (s,1H) ; 5.80 (d, J=2Hz, 1H).
4) RMN ¹³C CDCl₃ ; 50MHz :
   δ(ppm) :19.6 ; 19.9 ; 20.2 ; 20.7 ; 22.5 ; 22.8 ; 23.6 ; 24.0 ; 25.4 ; 25.5 ; 37.1 ; 37.4 ; 43.0 ; 43.3 ; 46.9 ; 47.3 ; 118.0 ; 118.2 ; 128.8 ; 129.2 ; 129.4 ; 130.0.

### 1.7 Préparation du tétramethyl-2,3,5,7 octane nitrile (IX) :

Dans un autoclave de 250 mL sont introduits successivement 15 g de nitrile VIII, 2g de palladium sur charbon (5%) et 100 mL de toluène sec. Le mélange est agité sous une pression normale d'hydrogène. L'évolution de la réaction est contrôlée par chromatographie gazeuse. Lorsque la réaction est terminée, le mélange réactionnel est filtré, lavé avec une solution d'acide chlorhydrique 5N puis concentré. Le résidu est distillé sous vide et 13.65g d'un liquide incolore sont obtenus, ce qui représente un rendement de 90%.
Eb = 100-102 °C / 800 Pa
1) IR : 2239, 2212, 1465, 1385, 1368, 1168 cm⁻¹
2) MS (70 eV) : m/e= 181(M+˙), 180, 166, 138, 125, 124, 110, 85, 71, 55, 43(100) uma
3) RMN ¹H : CDCl₃ ; 200 MHz *Mélange de 4 diastéréoisomères.*
   δ(ppm) :0.8 à 0.9 (m, 3H) ; 1.10 à 1.90 (m, 12H) ; 2.60 (m, 1H) ;
4) RMN ¹³C : CDCl₃ ; 50MHz : *Mélange de 4 diastéréoisomères*
   δ(ppm) : 14.5 ; 15.3 ; 15.6 ; 16.2 ; 16.4 ; 17.4 ; 17.6 ; 19.5 ; 19.7 ; 20.5 ; 20.9 ; 22.1 ; 22.4 ; 22.8 ; 23.4 ; 23.6 ; 23.9 ; 24.2 ; 25.4 ; 25.5 ; 25.6, 27.8 ; 27.9 ; 28.0 ; 31.0 ; 31.5 ; 32.2 ; 32.3 ; 33.5 ; 33.8 ; 33.9 ; 40.8 ; 41.6 ; 43.1 ; 43.6 ; 46.2 ; 46.7 ; 47.7 ; 48.0 ; 122.0 : 122.3 ; 122.7 ; 122.9.

### Exemple 2 : Evaluation olfactive du Verbetryle (II) et du Dihydroverbetryle™ (V) dans une composition parfumante.

Description olfactive :
Verbetryle (II) : vert, hespéridé, irisé, fleuri, un peu menthé.
Dihydroverbetryle™ (V) : vert, pamplemousse naturel, un peu menthé.

Des compositions parfumantes ont été créées (essais 2 et 3) dans lesquelles l'impact olfactif du Verbetryle™ et du Dihydroverbetryle™ a été examiné par comparaison avec une composition ne contenant pas le composé (essai 1).

| **Composants** | **Essai 1 (poids)** | **Essai 2 (poids)** | **Essai 3 (poids)** |
|---|---|---|---|
| Composition Bergamote | 100,00 | 100,00 | 100,00 |
| Essence de Carvi | 10,00 | 10,00 | 10,00 |
| Terpènes de citron | 100,00 | 100,00 | 100,00 |
| Mousse de Metra | 10,00 | 10,00 | 10,00 |
| Coumarine | 10,00 | 10,00 | 10,00 |
| Méthyl-Chavicol | 10,00 | 10,00 | 10,00 |
| Eugénol | 10,00 | 10,00 | 10,00 |
| Galaxolide | 55,00 | 55,00 | 55,00 |
| Essence de lavande | 25,00 | 25,00 | 25,00 |
| Essence de lavandin | 20,00 | 20,00 | 20,00 |
| Essence de Peppermint USA | 15,00 | 15,00 | 15,00 |
| Méthyl-dihydro-jasmonate | 70,00 | 70,00 | 70,00 |
| Composition Néroli | 100,00 | 100,00 | 100,00 |
| Octahydro-tetraméthyl-acétonaphtone | 100,00 | 100,00 | 100,00 |
| Essence d'orange | 200,00 | 200,00 | 200,00 |
| Essence de Spearmint | 55,00 | 55,00 | 55,00 |
| Composition Mandarine | 10,00 | 10,00 | 10,00 |
| Dipropylèneglycol | 100 | 70 | 70 |
| Verbetryle™ | 0 | 30 | 0 |
| Dihydroverbetryle™ | 0 | 0 | 30 |

Ces compositions ont été testées par exemple dans une base el douche nacrée à raison de 0,5% en poids. La composition de l'essai 2 apporte une note aromatique masculine, mentholée poivrée tandis que la composition de l'essai 3 apporte une note naturelle, pamplemousse avec une légère touche d'amertume par rapport à la composition de l'essai 1.

### Références

1) M. ERMAN ; Advances in thé Chemistry of Nitriles and Amides; Perfumer and Flavorist , 27, 30, (2002).
2) G.JONES; The Knoevenagel Condensation ; Organic Reactions 15, 204, (1967) John Wiley and Sons Ed.; New-York, London, Sidney.
3) A. HINNEN et J.DREUX ; Bull.Soc.Chim.Fr., 1964, p. 1492.

## Revendications

1. Composés de formule générale (I) ci-dessous : 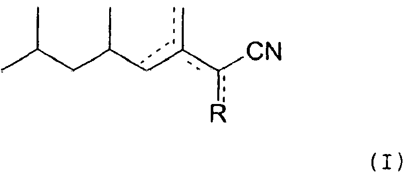 dans laquelle R est un atome d'hydrogène, un groupe méthyle, ou un groupe méthylène ; et dans laquelle au plus une des quatre lignes pointillées représente une double liaison carbone-carbone, ainsi que leurs énantiomères et leurs diastéréoisomères.

2. Composés selon la revendication **1** de formule générale (II) suivante : 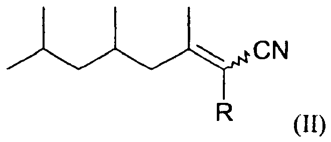 dans laquelle R est un atome d'hydrogène ou un groupe méthyle, leurs isomères Z ou E, leurs énantiomères.

3. Composés selon la revendication **1** de formule générale (III) suivante : 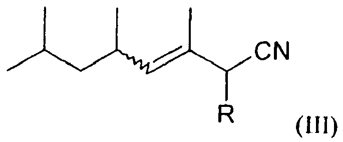 dans laquelle R est un atome d'hydrogène ou un groupe méthyle, leurs isomères Z ou E, leurs énantiomères et leurs diastéréoisomères.

4. Composés selon la revendication **1** de formule générale (IV) suivante : 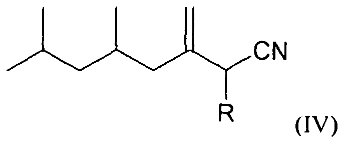 dans laquelle R est un atome d'hydrogène ou un groupe méthyle, leurs énantiomères et leurs diastéréoisomères.

5. Composés selon la revendication **1** de formule générale (V) suivante : 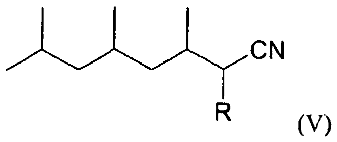 dans laquelle R est un atome d'hydrogène ou un groupe méthyle, leurs énantiomères et leurs diastéréoisomères.

6. Composé selon la revendication **1** de formule générale (VIII) suivante : 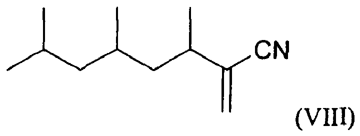 ses énantiomères et ses diastéréoisomères.

7. Composition, **caractérisée en ce qu'**elle comprend au moins un composé tel que défini dans l'une quelconque des revendications **1** à **6** sous forme d'un isomère ou d'un mélange d'isomères, d'un énantiomère ou d'un mélange d'énantiomères, ou d'un mélange racémique, ou d'un diastéréoisomère ou d'un mélange de diastéréoisomères.

8. Composition selon la revendication **7**, **caractérisée en ce qu'**elle comprend un mélange des composés de formule (II), (III), (IV) tels que définis dans les revendications **2** à **4**.

9. Composition selon la revendication **7**, **caractérisée en ce qu'**elle comprend au moins un des deux isomères Z et E du composé (II) tel que défini dans la revendication **2** ou un mélange desdits deux isomères.

10. Composition selon la revendication **7**, **caractérisée en ce qu'**elle comprend au moins un des quatre diastéréoisomères du composé (V) tel que défini dans la revendication **5** ou un mélange desdits quatre diastéréoisomères.

11. Composition selon l'une quelconque des revendications **7** à **10**, **caractérisée en ce qu'**elle comprend au moins un composé tel que défini dans l'une quelconque des revendications **1** à **6** et au moins une autre susbstance odorante.

12. Composition de parfumerie, notamment base ou concentré parfumant, eau de Cologne, eau de toilette ou parfum, **caractérisée en ce qu'**elle comprend au moins un composé tel que défini dans l'une quelconque des revendications **1** à **6** ou une composition selon l'une quelconque des revendications **7** à **11**.

13. Composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade, **caractérisée en ce qu'**elle comprend au moins un composé tel que défini dans l'une quelconque des revendications **1** à **6** ou une composition selon l'une quelconque des revendications **7** à **11**.

14. Produit d'entretien, notamment assouplissant, détergent, lessive, désodorisant d'ambiance, **caractérisé en ce qu'**il comprend au moins un composé tel que défini dans l'une quelconque des revendications **1** à **6** ou une composition selon l'une quelconque des revendications **7** à **11.**

15. Utilisation d'au moins un composé de formule (I) tel que défini dans l'une quelconque des revendications **1** à 6, ou d'une composition selon l'une quelconque des revendications **7** à **11**, comme agent odorant ou comme agent de masquage d'odeur ou comme agent de neutralisation d'odeur, optionnellement en association avec d'autres agents odorants.

16. Procédé de préparation des composés de formule (I) telle que décrite dans l'une quelconque des revendication 1 à 6, dans lequel une cétone, notamment la diméthyl-4,6 heptanone-2, et l'acide cyanoacétique ou un de ses esters, sont soumis à une condensation de Knoevenagel.

## Patentansprüche

1. Verbindungen der nachfolgenden allgemeinen Formel (I): 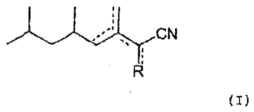 worin R ein Wasserstoffatom, eine Methylgruppe oder eine Methylengruppe ist, und worin höchstens eine der vier gepunkteten Linien für eine Kohlenstoff-Kohlenstoff-Doppelbindung steht, sowie deren Enantiomere und Diastereomere.

2. Verbindungen nach Anspruch 1 der folgenden allgemeinen Formel (II): 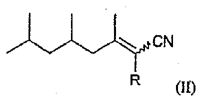 worin R ein Wasserstoffatom oder eine Methylgruppe ist, deren Z- oder E-Isomere, deren Enantiomere.

3. Verbindungen nach Anspruch 1 der nachfolgenden allgemeinen Formel (III): 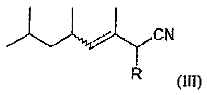 worin R ein Wasserstoffatom oder eine Methylgruppe ist, deren Z- oder E-Isomere, deren Enantiomere und deren Diastereomere.

4. Verbindungen nach Anspruch 1 der nachfolgenden allgemeinen Formel (IV): 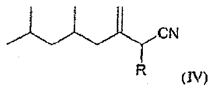 worin R ein Wasserstoffatom oder eine Methylgruppe ist, deren Enantiomere und deren Diastereomere.

5. Verbindungen nach Anspruch 1 der nachfolgenden allgemeinen Formel (V): 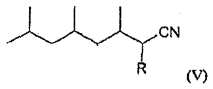 worin R ein Wasserstoffatom oder eine Methylgruppe ist, deren Enantiomere und deren Diastereomere.

6. Verbindung nach Anspruch 1 der folgenden allgemeinen Formel (VIII): 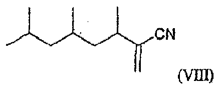 ihre Enantiomere und ihre Diastereomere.

7. Zusammensetzung, **dadurch gekennzeichnet, dass** sie mindestens eine Verbindung wie in einem der Ansprüche 1 bis 6 definiert enthält und zwar in Form eines Isomers oder eines Gemischs von Isomeren, eines Enantiomers oder eines Gemischs von Enantiomeren oder eines racemischen Gemischs oder eines Diastereomers oder eines Gemischs von Diastereomeren.

8. Zusammensetzung nach Anspruch 7, **dadurch gekennzeichnet, dass** sie ein Gemisch der Verbindungen der Formeln (II), (III), (IV) wie in den Ansprüchen 2 bis 4 definiert enthält.

9. Zusammensetzung nach Anspruch 7, **dadurch gekennzeichnet, dass** sie mindestens eines der beiden Z- und E-Isomere der Verbindung (II) wie in Anspruch 2 definiert oder ein Gemisch dieser beiden Isomere enthält.

10. Zusammensetzung nach Anspruch 7, **dadurch gekennzeichnet, dass** sie mindestens eines der vier Diastereomere der Verbindung (V) wie in Anspruch 5 definiert oder ein Gemisch der genannten vier Diastereomere enthält.

11. Zusammensetzung nach einem der Ansprüche 7 bis 10, **dadurch gekennzeichnet, dass** sie mindestens eine Verbindung wie in einem der Ansprüche 1 bis 6 definiert und mindestens eine weitere riechende Substanz enthält.

12. Duftzusammensetzung, insbesondere Parfümgrundstoff oder -konzentrat, Kölnisch Wasser, Toilettenwasser oder Parfüm, **dadurch gekennzeichnet, dass** sie mindestens eine Verbindung wie in einem der Ansprüche 1 bis 6 definiert oder eine Zusammensetzung nach einem der Ansprüche 7 bis 11 enthält.

13. Kosmetische Zusammensetzung, insbesondere Gesichts- oder Körpercreme, Puder, Haar- oder Körperöl, Shampoo, Haarlotion, Badesalz, Badeöl, Duschgel, Badegel, Seife, Körperantiperspirant, Körperdeodorant, Lotion, Rasiercreme, Rasierseife, Creme, Zahnpasta, Duschbad, Pomade, **dadurch gekennzeichnet, dass** sie mindestens eine Verbindung wie in einem der Ansprüche 1 bis 6 definiert oder eine Zusammensetzung nach einem der Ansprüche 7 bis 11 enthält.

14. Pflegemittel, insbesondere Weichspüler, Detergens, Waschmittel, Raumdesodorant, **dadurch gekennzeichnet, dass** es mindestens eine Verbindung wie in einem der Ansprüche 1 bis 6 definiert oder eine Zusammensetzung nach einem der Ansprüche 7 bis 11 enthält.

15. Verwendung mindestens einer Verbindung der Formel (I) wie in einem der Ansprüche 1 bis 6 definiert oder einer Zusammensetzung nach einem der Ansprüche 7 bis 11 als Duftmittel oder als Geruchsmaskierungsmittel oder als Mittel zur Neutralisation von Gerüchen, optional zusammen mit anderen Duftmitteln.

16. Verfahren zur Herstellung der Verbindungen der Formel (I) wie in einem der Ansprüche 1 bis 6 definiert, wobei ein Keton, insbesondere 4,6-Dimethylheptanon-2, und Cyanoessigsäure oder einer ihrer Ester einer Knoevenagel-Kondensationsreaktion unterworfen werden.

## Claims

1. Compounds of general formula (I) below: 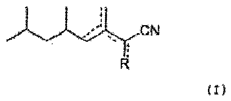 in which R is a hydrogen atom, a methyl group or a methylene group, and in which at least one of the dotted lines represents a carbon-carbon double bond, together with their enantiomers and diastereoisomers.

2. Compounds according to claim 1 of general formula (II) below: 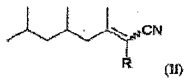 in which R is a hydrogen atom or a methyl group, their *Z* or *E* isomers or their enantiomers.

3. Compounds according to claim 1 of general formula (III) below: 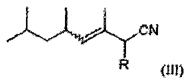 in which R is a hydrogen atom or a methyl group, their *Z* or *E* isomers, their enantiomers and their diastereoisomers.

4. Compounds according to claim 1 of general formula (IV) below: 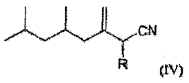 in which R is a hydrogen atom or a methyl group, their enantiomers and their diastereoisomers.

5. Compound according to claim 1 of general formula (V) below: 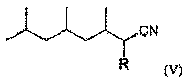 in which R is a hydrogen atom or a methyl group, their enantiomers and their diastereoisomers.

6. Compound according to claim 1 of general formula (VIII) below: 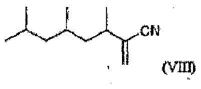 its enantiomers and its diastereoisomers.

7. Composition **characterised in that** it comprises at least one compound as defined in any one of claims 1 to 6 in the form of an isomer or a mixture of isomers, an enantiomer or a mixture of enantiomers, or a racemic mixture, or a diastereoisomer or a mixture of diastereoisomers.

8. Composition according to claim 7, **characterised in that** it comprises a mixture of the compounds of formulae (II), (III), (IV) as defined in claims 2 to 4.

9. Composition according to claim 7, **characterised in that** it comprises at least one of the two Z and E isomers of compound (II) as defined in claim 2 or a mixture of these two isomers.

10. Composition according to claim 7, **characterised in that** it comprises at least one of the four diastereoisomers of compound (V) as defined in claim 5 or a mixture of these four diastereoisomers.

11. Composition according to any one of claims 7 to 10, **characterised in that** it comprises at least one compound as defined in any one of claims 1 to 6 and at least one other odourising substance.

12. Perfumery composition, in particular a perfumery base or concentrate, eau de cologne, toilet water or perfume, **characterised in that** it comprises at least one compound as defined in any one of claims 1 to 6 or a composition according to any one of claims 7 to 11.

13. Cosmetic composition, in particular face and body cream, talcum powder, hair or body oil, shampoo, hair lction, bath salt, bath oil, shower gel, bath gel, toilet soap, body antiperspirant, body deodorant, lotions, shaving cream, shaving soap, toothpaste, mouthwash, ointment, **characterised in that** it comprises at least one compound as defined in any one of claims 1 to 6 or a composition according to any one of claims 7 to 11.

14. Toiletry product, in particular a softener, detergent, cleaning agent, environmental deodoriser, **characterised in that** it comprises at least one compound as defined in any one of claims 1 to 6 or a composition according to any one of claims 7 to 11.

15. Use of at least one compound of formula (I) as defined in any one of claims 1 to 6, or a composition according to any one of claims 7 to 11 as a deodourising agent or as an odour masking agent or as an odour neutralising agent, optionally in association with other odourising agents.

16. Process for the preparation of compounds of formula (I) as described in any one of claims 1 to 6, in which a ketone, in particular dimethyl-4,6-heptanone-2 and cyanoacetic acid or one of its esters are subjected to a Knoevenagel condensation.
